# EUROPEAN PATENT SPECIFICATION

(11) **EP 1 090 133 B1**
(45) Date of publication and mention of the grant of the patent: **12.08.2009**
(21) Application number: 99926204.1
(22) Date of filing: 21.06.1999
(51) Int. Cl.: C12N 15/82, C12N 15/12, A61K 38/17

(54) **PRODUCTION OF IL-10 IN NON-FOOD CROP PLANT BIOREACTOR**
HERSTELLUNG VON IL-10 IN EINEN PFLANZENBIOREAKTOR AUS PFLANZEN, DIE NICHT ZUR ERNÄHRUNG VERWENDET WERDEN
PRODUCTION DE IL-10 DANS UN BIOREACTEUR DE PLANTES CULTIVEES NON ALIMENTAIRES

(30) Priority: 22.06.1998 US 102050
(43) Date of publication of application: 11.04.2001
(73) Proprietor: HER MAJESTY THE QUEEN IN RIGHT OF CANADA, as represented by the Minister of Agriculture and Agri-Food, Ontario N5V 4T3 (CA); Queen's University, Kingston, Ontario K7L 3N6 (CA); The John P. Robarts Research Institute, London, Ontario N6A 5K8 (CA); London Health Sciences Center, London, Ontario N6A 5A5 (CA)
(72) Inventor: BRANDLE, Jim, London, Ontario N6A 3Y5 (CA); DAVIES, Peter, L., Kingston, Ontario K7M 2M8 (CA); KENWARD, Kimberley, D., London, Ontario N6A 1M1 (CA); MENASSA, Rima, London, Ontario N5X 1W4 (CA); JEVNIKAR, Tony, London, Ontario N6H 3Y8 (CA); DELOVITCH, Terry, London, Ontario N6G 2S6 (CA)
(74) Representative: Main, Malcolm Charles
(86) International application number: PCT/CA1999/000577
(87) International publication number: WO 1999/067401

(56) References cited:
- WO-A-93/03161
- CA-A- 2 188 220
- SPURGEON D.: "Tobacco could be used to produce interleukin 10" BRITISH MEDICAL JOURNAL, vol. 319, no. 7203, 17 July 1999 (1999-07-17), pages 143-143, XP002127522
- LEVINE ET AL.: "High level expression and refolding of mouse interleukin 4 synthesized in E. coli." J. BIOL. CHEM., vol. 270, no. 13, 31 March 1995 (1995-03-31), pages 7445-7452,
- SYTO ET AL.: "Structural and biological stability of the human interleukin 10 homodimer" BIOCHEMISTRY, vol. 37, 11 July 1998 (1998-07-11), pages 16943-16951,
- VIEIRA ET AL.: "Isolation and expression of human cytokine synthesis inhibitory factor cDNA clones: homology to Epstein-Barr virus open reading frame BCRFI" PNAS, vol. 88, February 1991 (1991-02), pages 1172-1176,
- ZDAVOV ET AL.: "Crystal structure of interleukin-10 reveals the functional dimer with an unexpected topological similarity to interferon gamma" CURRENT BIOLOGY-STRUCTURE, vol. 3, 15 June 1995 (1995-06-15), pages 591-601,
- WERNER ET AL.: "Appropriate mammalian expression systems for biopharmaceuticals" ARZNEIM. -FORSCH./DRUG RES., vol. 48, no. II-8, 1998, pages 870-880,
- EDELBAUM ET AL.: "Expression of active human interferon-beta in transgenic plants" J. INTERFERON RES., vol. 12, 1992, pages 449-453,
- MENASSA ET AL.: "Therapeutic effectiveness of orally administered transgenic low-alkaloid tobacco expressing human interleukin-10 in a mouse model of colitis" PLANT BIOTECHNOLOGY JOURNAL, vol. 5, 2006, pages 50-59,

## Description

The present invention relates to the use of a non-food crop plant as a bioreactor. More specifically this invention relates to the expression of IL-10 for oral administration using non-food crop plants.

### BACKGROUND OF THE INVENTION

Full citations for references appear at the end of the examples section.

Numerous plants have proven themselves to be amenable to transformation with heterologous genes and for some time tobacco has been the model system for plant transformation. Despite the fact that crop-protection focused biotechnologies have not found application in non-food crop plant production, a major role does remain for such plants as bioreactors. An example of a non-food crop plant is tobacco, which is capable of producing high levels of soluble protein (fraction 1 protein, FIP; Woodleif et al 1981) and pilot systems have been developed to purify this fraction for use as a high protein dietary supplement (Montanari et al 1993).

Expression of mammalian genes in several plants including tobacco and *Arabidopsis* has been recognized as efficient, low cost, non-sterile bioreactors for the production of proteins valuable to both medicine and industry (Ma and Hein 1995). Recently evidence was presented that demonstrated that the four chains of the secretory immunoglobulin were properly expressed and assembled in plants and that the antibody was fully functional (Ma et al 1995). Furthermore, bacterial (Haq et al 1995), and viral (Mason et al 1996) antigens produced in transgenic tobacco and potato effectively immunized mice when the transgenic potato was administered orally. However, prior to the administration of plant tissue obtained from transgenic tobacco, the proteins had to be partially purified. Clearly steps involving processing are undesirable if ease of oral administration is to be maximized as well as minimizing any associated costs for production.

From both a regulatory and public safety stand point non-food crop plants are ideal species for the transgenic production of biologically active proteins. Non-food crop plants minimize the risk of accidental leakage of transgenic plant material expressing genes for biologically active proteins into the humane food chain. Other plant bioreactor systems based on canola (Rooijen et al. 1995), potato (Manson et al 1996), rice and cassava (Ma and Hein 1995) do not offer this advantage. Furthermore, non-food crop plants can be selected so that production in areas where there are no naturally occurring wild species further minimizes the risk of gene leakage to the local flora, an example of this would be to grow tobacco in regions where tobacco does not overwinter, such as Canada. With any non-food crop plant, transgenic proteins can be produced using any tissue or organ of the plant. However if protein production is based on leaves, not seeds or tubers, and when coupled with the fact that the leaves are harvested before flowering there is virtually no risk of uncontrolled bioreactor plants occurring in future crop seasons.

However, many non-food crop plants contain high levels of secondary plant products making plant tissue obtained from these plants unsuitable for direct oral administration. Earlier studies have described the administration of tobacco-derived proteins to mice, however, the proteins were in a partially purified form. For example the study by Mason et al (1996) involved the direct oral administration of viral antigens expressed in potato tuber and tobacco. The potato tuber samples were directly fed to mice, yet the antigen, when obtained from tobacco, had to be partially purified using sucrose gradients prior to administration to mice.

The breeding of low alkaloid-containing tobacco plants has been reported (Chaplin 1977), however, use of such a plant as a bioreactor has not been suggested. Recently, specific alteration of nicotine levels within tobacco, by either over expression (i.e. increasing) or antisense expression (decreasing) of putrescine N-methyltransferase, a rate limiting enzyme involved in the nicotine biosynthetic pathway, has been suggested (U.S. 5,260,205, issued November 9, 1993 and U.S. 5,369,023, issued November 29, 1994; inventors Nakatani and Malik). However, these methods are directed to the alteration of nicotine levels so that the levels of other alkaloids that affect the flavours and aroma of tobacco are not modified in any manner. These plants would not be suitable for use as a non-food crop plant as describe herein, since the levels of only a select group of alkaloids have been reduced. No nicotine free plants were actually produced, nor was the use of these transgenically modified tobacco plants, as a bioreactor for the synthesis of proteins of interest, suggested. However, such methods may be used and augmented in order to produce non-food crop plants where the total alkaloid level is reduced. Such an approach may be useful for the production of low alkaloid plants as a bioreactor for the synthesis of proteins of interest as contemplated by this invention. CA 2188 220 discloses the production of IL-4 in tobacco.

Thus there is a need to provide a non-food crop plant capable of being used to prepare transgenic proteins of interest suitable for oral administration.

### SUMMARY OF THE INVENTION

The present invention relates to the production of a protein of interest using a non-food crop plant.

According to the present invention there is provided a method for the preparation of IL-10 suitable for oral administration within a non-food crop plant comprising, transforming the non-food crop plant with a suitable vector containing a nucleic acid encoding human IL-10 and appropriate regulatory regions to ensure expression of the nucleic acid within the non-food crop plant, such that the non-food crop plant is characterized as being non-toxic, non-addictive, palatable, and requiring little or no processing prior to oral administration , selecting non-food crop plants that express human IL-10 to obtain transformed non-food crop plants; growing and harvesting said transformed plants and obtaining plant tissu comprising human IL-10 from said transformed plants.

This invention further relates to the above method wherein the non-food plant is
characterized in having a low alkaloid level. This invention also includes methods utilizing a plant characterized in having a low nicotine level.

This invention also describes the above method wherein the protein of interest includes pharmaceutically active proteins, such as growth regulators, insulin, interferon and related compounds, interleukins, growth hormone, erythropoietin, G-CSF, GM-CSF, hPG-CSF, M-CSF, Factor VIII, Factor IX, tPA, antibodies, antigens and any combinations and derivatives thereof.

Furthermore, this invention includes the above method wherein a suitable vector contains a gene of interest along with appropriate regulatory regions to ensure expression of the IL-10 within desired plant tissues.

This invention also provides for compositions comprising a tissue from a non-food crop containing a transgenic IL-10.

Another aspect of an embodiment of this invention is a method for the treatment of a medical aliment by administering a suitable amount of the above composition comprising a tissue from a non-food crop plant containing as a pharmaceutically active component a transgenic IL-10.

This invention is also directed to a method for preparing human IL-10 within a non-food crop plant comprising;
i) selecting a non-transformed tobacco plant comprising from about 0.2% to about 20% of the total alkaloid of delgold and characterized as being non-toxic, non-addictive, and palatable;
ii) transforming said non-transformed tobacco plant with a vector comprising a gene capable of encoding said human IL-10 and 5' and 3' regulatory regions to ensure expression of the gene within said non-transformed tobacco plant to produce a transformed plant;
   and
iii) obtaining human IL-10 from said transformed plant.

Furthermore, the method for the preparation of IL-10 may comprise
i) transforming a plant with a vector comprising a gene encoding IL-10, operatively linked with 5' and 3' regulatory regions;
ii) selecting transformed plants that contain IL-10;
iii) growing and harvesting selected plants; and
iv) extracting IL-10 from said selected plants.

In the present invention said IL-10 is human IL-10. Optionally, the vector may comprise sequences that enhance expression of said IL-10, such as an ER retention signal. Furthermore, the vector may also encode a proteolytic cleavage site, an affinity tag, or a combination thereof.

Although the present invention is exemplified by the preparation of transgenic proteins of interest using tobacco, in practice any non-food crop plant may be used for the preparation of a protein of interest following the methods as disclosed herein.

### BRIEF DESCRIPTION OF THE DRAWINGS

These and other features of the invention will become more apparent from the following description in which reference is made to the appended drawings wherein:

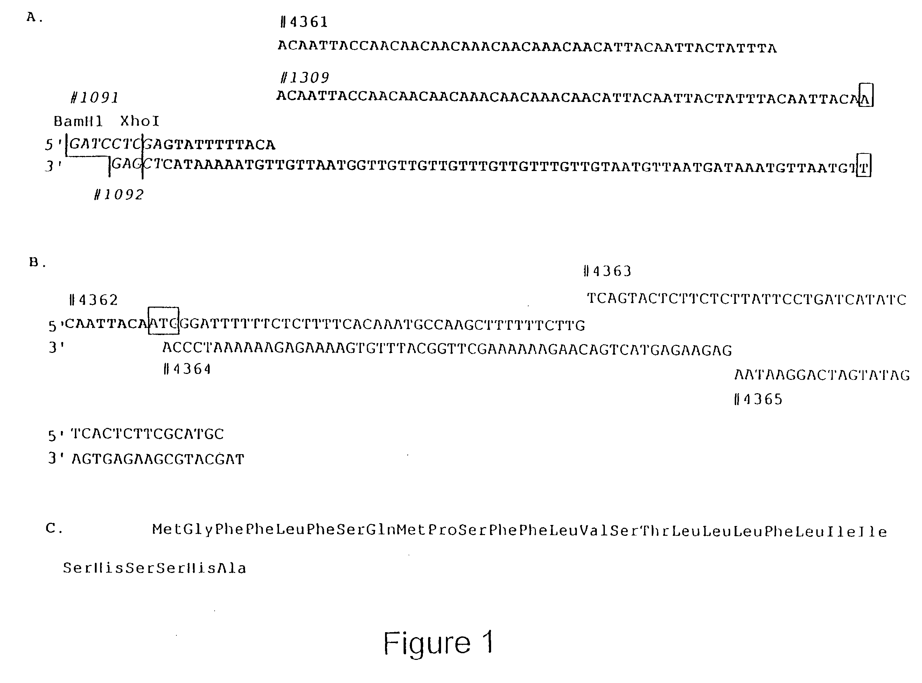

Figures 1-4 are for comparative purpose.
   - **FIGURE 1**: shows the oligonucleotide cassettes for integration of TMV 5'-untranslated region and PR-1b transit peptide sequences into transgene constructs. Figure 1(A) the TMV leader sequence. Figure 1(B) the PR-1b signal peptide sequence. Figure 1(C) the amino acid translation of encoded signal peptide. Identity, sequence, and position of each oligonucleotide within the cassettes are indicated.
   TMV sequence is marked in bold. PR-1b signal peptide sequence in plain letters and additional sequence in italics. Nucleotides constituting all or part of the initiation codon are present within boxes. Oligonucleotides #1091 (SEQ ID NO:1); # 1092 (SEQ ID NO:4); and # 1093 (SEQ ID NO:2) represent the TMV oligo set. Oligonucleotides #1091, #1092 (SEQ ID NO:1 and 4), and #4361 through #4365 (SEQ ID NOs: 3, 5, 6. 8 and 9 respectively) represent the TMV-PR oligo set.
   - **FIGURE 2**: displays the map of the T-DNA region of pCDX-TL-MAFPII
   - **FIGURE 3**: displays the map of the T-DNA region of pMON-TL-MAFPII
   - **FIGURE 4**: is a Western blot showing Type II AFP accumulation in field plants.
   Western blot analysis of total soluble protein (2.5 µg) extracts from R1 generation Type II AFP transgenic plants in which the AFP was targeted to accumulate in the extracellular space. Samples are designated by number to indicate the plot from which it was collected and by letter to indicate the transformed parent plant from which the R1 plants were descended. Records at the Delhi station indicate that A plants were descended from parent II4c²-#10, B plants from II4c²-#1, C from pII3a-#7, and D plants were wild-type controls (4D). Total soluble protein extract from a field-grown wild type plant was included in the analysis as a negative control and mixed with mature Type II AFP (25 ng) purified from sea raven sera to generate the positive control (AFP).

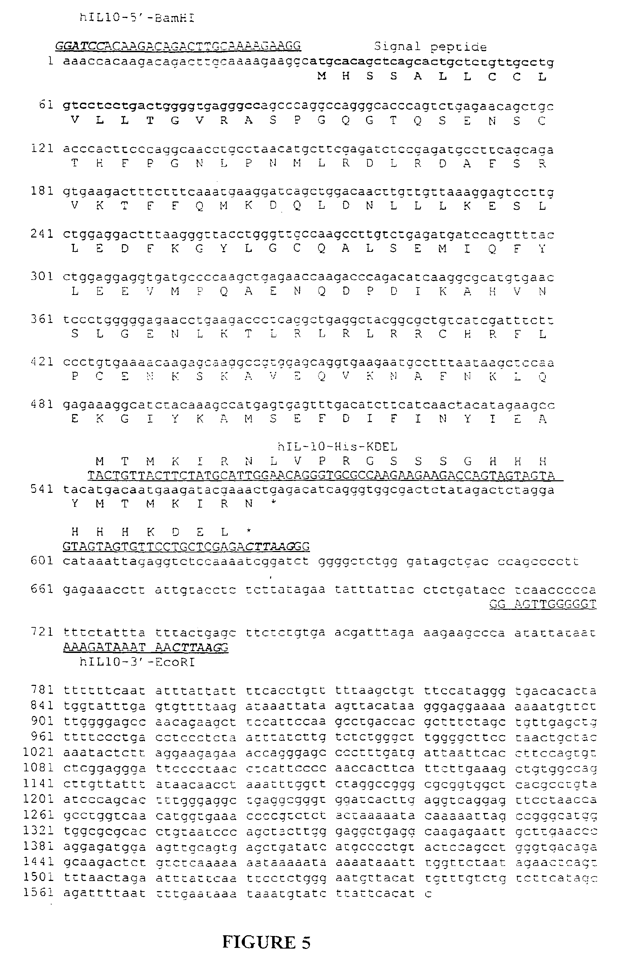

   - **FIGURE 5**: shows the cDNA sequence of human IL-10. The DNA sequence is denoted in small letters, the encoded protein sequence is denoted in capital letters below the nucleotide sequence with the one letter amino acid code. The signal peptide sequence is boxed and shaded. Oligonucleotides used to amplify hIL-10 sequences or to fuse other sequences to the hIL-10 gene are shown in capital letters underlined by an arrow. Restriction sites introduced by PCR are shaded. Encoded amino acids are shown above the hIL-10-His-KDEL oligonucleotide sequence.

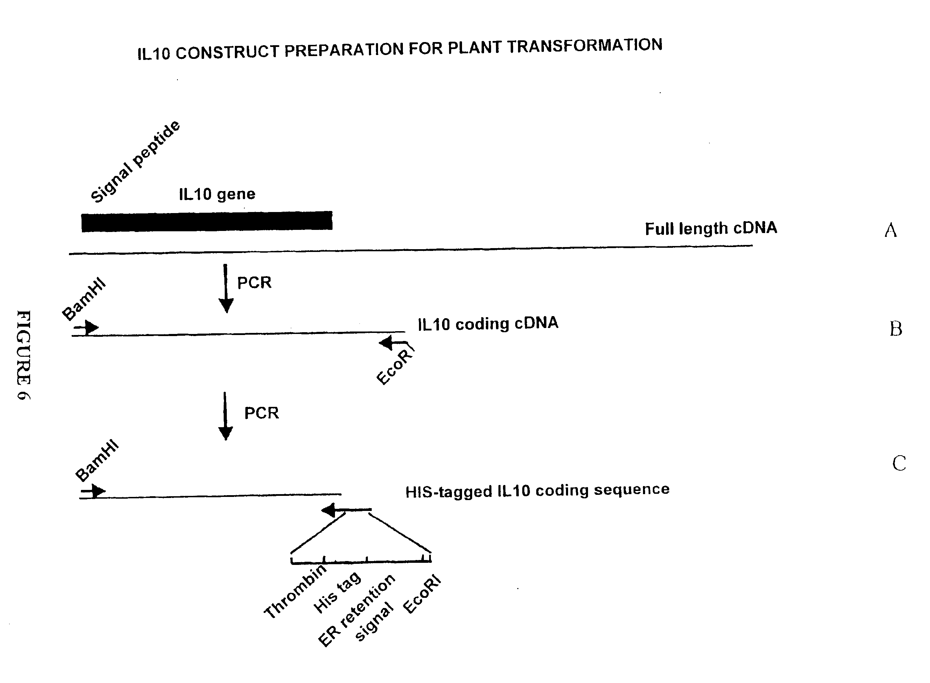

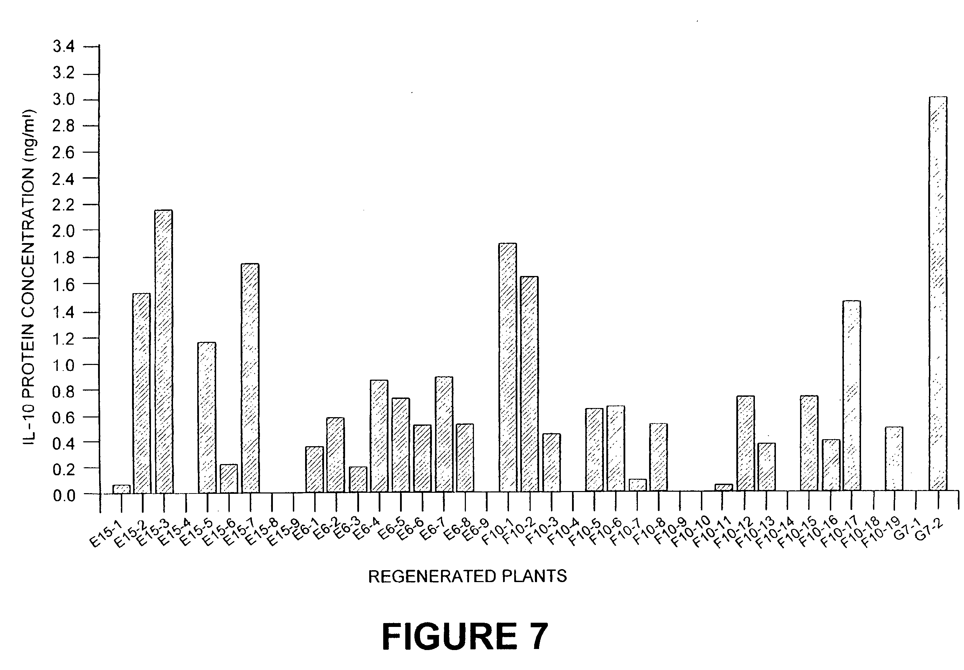

   - **FIGURE 6**: shows a schematic depiction of three IL-10 constructs used in plant transformation as described herein. Figure 6 (A) shows the full length, 1601 bp DNA of IL-10. Transformed plants comprising this construct are designated as "F" plants in Figure 7. Figure 6 (B) shows a truncated IL-10 sequence comprising nucleotides 4-732, with only a portion of the 3' UTR (168 bp). Transformed plants comprising this construct are designated as "E" plants in Figure 7. Figure 6 (C) shows an IL-10 construct with the 3' UTR portion removed, and a thrombin recognition sequence, a His tag and a KDEL sequence added to the 3' end of the IL-10 sequence. The region containing the thrombin-His tag and KDEL sequence is magnified to show the order of those sequences with respect to the hIL-10 gene. Transformed plants comprising this construct are designated as "G" plants in Figure 7. The hIL-10 coding region is depicted by a dark shaded box, the signal peptide is shown by a light shaded box, and the cDNA is shown by a straight line. Oligonucleotides are represented by short arrows.
   - **FIGURE 7**: shows the IL-10 protein levels within transgenic plants as determined by ELISA. The protein concentration was determined and compared against a hIL-10 standard curve. Individual transformed plants were analyzed. Plants denoted "E" were transformed with hIL-10 construct containing coding sequences and a short 3' UTR; plants denoted "F" were transformed with hIL-10 construct containing coding sequences and the complete 3' UTR; plants denoted "G" were transformed with hIL-10 construct containing coding sequences fused to thrombin-His tag-KDEL sequence.

### DESCRIPTION OF PREFERRED EMBODIMENT

From both a regulatory and public safety stand point non-food crop plants are ideal species for the transgenic production of biologically active proteins since the risk of leakage of transgenic plant material into the human food chain is negligible. Other plant bioreactor systems based on food crops do not offer this advantage. Even though non-food crop plants are ideal species for use as bioreactors from both a regulatory and public safety point of view, there a several obstacles that must be overcome prior to their use as bioreactors.

A major problem with the use of non-food crop plants, for example tobacco, as bioreactors is that these plants may contain undesirable secondary plant products. Secondary plant products are constituents that are generally toxic or reduce the palatability of the plant tissue or that are addictive in nature. This is a significant concern since one of the benefits of preparing proteins of interest within a plant is that large quantities of protein may be required for administration (Ma and Hein, 1995), therefore any toxic, addictive, or otherwise non-desirable products should be avoided within the plant tissue. For example, tobacco plants contain high levels of secondary plant products such as nicotine and related alkaloids, making the plant tissue unsuitable for the direct oral administration. Earlier studies have described the administration of tobacco-derived proteins to mice, however, the proteins were in a partially purified form. For example, the study by Mason et al (1996) involved the direct oral administration of viral antigens expressed in potato tuber and tobacco. The potato tuber samples were directly fed to mice, yet the antigen, when obtained from tobacco, had to be partially purified using sucrose gradients prior to administration to mice. It is desirable to prepare transgenic proteins within non-food crop plants that permit the direct use of plant tissue for oral administration, or following minimal processing.

By "non-food crop plant" it is meant a plant that has traditionally not been used, or grown for food purposes. Examples of non-food crop plants include, but are not limited to, ornamental plants, tobacco, hemps, weeds, and the like and trees. It is contemplated that such non-food crops plants may be used for the production of a protein of interest. In order to that the non-food crop plant to be used as a bioreactor for the production of a transgenic-expressed protein of interest suitable for direct oral administration, or requiring only minimal processing prior to oral administration, such a non-food crop plant is characterized as being:
a) is non-toxic;
b) is palatable following minimal or no processing; and
c) is characterized in having low levels of non-desirable secondary plant products such as alkaloids, nicotine, or the like.

However, if it is desired that the protein of interest is to not be orally administered, than any non-food crop plant may be used for the production of a protein of interest following the methods of the present invention as described herein. In such applications where there is no need for orally administering the protein of interest, the protein of interest may be combined with other nucleic acid sequences that are useful for the purification of the protein of interest in order that the protein be suitable for further use.

Use of the terms "low nicotine" and "low alkaloid" with reference to plants, such as, but not limited to tobacco, means a plant that contains a significantly lower concentration of alkaloids when compared with a similar plant comprising regular alkaloid content. For example, which is not to be considered limiting in any manner, a low alkaloid tobacco plant may be defined as a plant that contains less than from about 0.2% to about 70 % of the total alkaloids present in plants comprising a regular level of alkaloids. More preferably a low-alkaloid plant is one that contains about 0.2% to about 10% of the total alkaloids present in plants comprising a regular level of alkaloids. An example of a low alkaloid plant, that comprises about 2.8% of the total alkaloid level of a regular tobacco, is 81 V-9 (see Table 1, Example 4). Similarly, a low nicotine tobacco plant is one that contains a significantly lower concentration of nicotine when compared with a similar plant comprising regular nicotine content. For example, which is not to be considered limiting in any manner, a low nicotine tobacco plant may be defined as a plant that contains less than from about 0.2% to about 70 % of the total nicotine present in plants comprising a regular level of nicotine. More preferably a low-nicotine plant is one that contains from about 0.2% to about 10% of the total nicotine present in plants comprising a regular level of nicotine. An Example of a low nicotine plant, that comprises about 2.6% of the total nicotine level of regular tobacco, is 81V-9 (Table 1. Example 4).

Such low alkaloid or low nicotine plants may be obtained through conventional breeding programs (e.g. Chaplin, 1977), through selective down regulation of undesired genes (e.g. U.S. 5,260,205, issued November 9, 1993 and U.S. 5,369,023, issued November 29, 1994; inventors Nakatani and Malik), or by any other means e.g. mutagenesis followed by selection of desired traits. However, as is known to one of skill in the art, the ultimate nicotine or alkaloid level may still depend upon the environment under which the plant is grown.

By "minimal processing" it is meant the rapid processing of transgenic proteins produced within non-food crop plants. Such rapid processing could involve, but is not limited to, methods as those employed for the preparation of FIP as disclosed in Woodleif et al (1981). These include the aqueous extraction of soluble protein from green tobacco leaves by precipitation with KHSO₄, following removal of chloroplastic debris. Other methods may include large scale maceration and juice extraction in order to permit the direct use of the extract. If mechanically extracted at the filed site in the presence of water, this extract may be further processed prior to administration. Protein compositions prepared from minimally processed plant tissues comprise the transgenic protein of interest along within plant-derived components including cellular components and other soluble materials.

By "suitable vector" it is meant a vector comprising a gene of interest that is capable of being expressed within plant tissue. Such a vector may also include ubiquitous or tissue specific promoters and other 5' and 3' regulatory elements as would be known to one of skill in the art. Other elements that may be included with this vector include sequences for targeting the protein of interest to the cytosol or secretory pathway such as, but not limited to, the C-terminal KDEL sequence, an endoplasmic reticulum retention motif (Schouten et al 1966). Other retention signals, as would be known to one of skill in the art, may also be used for this purpose. Furthermore, such a vector may include marker genes for the detection of expression within the transgenic plant, and other sequences that aid in the purification of a protein of interest encoded by the gene of interest, including proteolytic cleavage, column chromatography and the like. An example, which is not to be considered limiting in any manner, of a sequence that aids in the purification of a protein of interest includes an affinity tag, for example, sequences that encode a His tag. However, it is to be understood that other affinity tags, as are known within the art, may also be used for the purpose of purification. An example, which is not to be considered limiting in any manner of a sequence that aids in proteolytic cleavage, and is used to separate the protein of interest from other translated sequences such as a KDEL sequence includes, for example, a sequence encoding a thrombin cleavage site. Again, it is to be understood that other proteolytic cleavage sites may also be used as would be evident to one of skill in the art.

By "protein of interest" it is meant any protein that is to be expressed in a transformed plant. Such proteins may include, but are not limited to, pharmaceutically active proteins, for example IL-1, IL-2, IL-3 ... IL-12, EPO, CSF, including G-CSF. GM-CSF, hPGCSF, M-CSF, Factor VIII, Factor IX, tPA, hGH, receptors, receptor agonists, antibodies, neuropolypeptides, insulin, vaccines, growth factors, growth regulators, antigens, their derivatives and the like.

By "oral administration" it is meant the administration of a tissue or organ of a non-food crop plant, for example the leaf, root, fruit etc with minimal or no prior processing. Such tissues or organs may be provided in the form of a salad or the like along with other pharmaceutical ingredients, or ingredients to increase palatability, if desired. It is also contemplated that minimal processing of the tissue or organ may take place prior to administration of the extract. For example, proteins secreted within the extracellular space of leaf tissues could be readily obtained using vacuum or centrifugal extraction, or tissues could be extracted under pressure by passage through rollers or grinding or the like to squeeze or liberate the protein free from within the extracellular space. Minimal processing could also involve preparation of crude extracts of soluble proteins, since these preparations would have negligible contamination from secondary plant products.

Bv "medical aliment" it is meant a defined medical condition that can be treated a specific pharmaceutical suitable for the treatment of the condition. Examples of such medical ailments and their corresponding suitable pharmaceutical include, but are not limited to: diabetes and the administration of IL-4 or insulin or a combination thereof; conditions related to blood clotting and the administration of Factor VIII, Factor IX or tPA or combinations thereof; medical conditions requiring the stimulation of progenitor cells to monocytes/macrophages and the administration of G-CSF, GM-CSF, hPG-CSF, M-CSF or combinations thereof; viral infections and the administration of interferons e.g. interferon-α, interferon-β, interferon-τ.

Several methods have been proposed for the removal of nicotine from tobacco. however, these processes typically involve the treatment of post harvest-tissue. For example the use of solvents (EP 10,665, published May 14, 1980; inventors Kurzhalz and Hubert), or potassium metabisulphite, potassium sulphate and nitrate (U.S. 4,183,364, issued January 8, 1980; inventor Gumushan) have been proposed as methods for removing nicotine from tobacco leaves. All of these treatments are designed to maintain the flavour and aroma of the tobacco, and these methods involve extensive post-harvest processing and are therefore not suitable for the preparation of products as described in this invention.

Specific alteration of nicotine levels within tobacco, by either over expression (i.e. increasing) or antisense expression (decreasing) of putrescine N-methyltransferase, a rate limiting enzyme involved in the nicotine biosynthetic pathway, has been suggested (U.S. 5,260,205, issued November 9, 1993 and U.S. 5,369,023, issued November 29, 1994; inventors Nakatani and Malik). These methods are directed to the alteration of nicotine levels so that the levels of other alkaloids that affect the flavours and aroma of tobacco are not modified in any manner. No nicotine free plants were actually produced, nor was the use of these transgenically modified tobacco plants, as a bioreactor for the synthesis of proteins of interest, suggested. However, such methods, if modified to reduce total plant alkaloid levels, may be used in order to produce non-food crop plants wherein total alkaloid levels are reduced.

In order to establish the efficacy of transgene expression in a low alkaloid plant, for example a nicotine-free tobacco such as but not limited to 81V-9, and ensure soluble protein production, several proteins of interest were expressed within a low alkaloid plant. These proteins include IL-4, IL-10, and a Type II antifreeze protein (AFP), however, it is to be understood that other proteins of interest may be also used to transform a low alkaloid, or low nicotine plant according to the methods of this invention.

IL-4 is a growth and differentiation factor for T-cells. In particular, IL-4 promotes the development of the subset of T helper (Th2) cells from native T cells upon antigen stimulation. IL-4 producing Th2 cells generally protect against the onset of many organ-specific autoimmune diseases, including type 1 diabetes. Thus the availability of an abundant source of IL-4 may prove invaluable for the immunotherapy of diabetes. Transgenic low-nicotine tobacco plants can be used in direct feeding studies to determine the efficacy of the recombinantly produced protein.

Crohn's disease is a chronic inflammatory bowel disease with frequent remissions and exacerbations, and several extra-GI manifestations. Lesions are typically discontinuous and can occur anywhere within the GI tract. The most common locations are ileum and colon, and formation of strictures and fistulas are common. In contrast, ulcerative colitis is contiguous and characteristically affects the colon. While the etiology of both these inflammatory bowel diseases remain unknown, there is data demonstrating the participation of immune cells in these conditions. Cytokines may direct the intensity, extent, and eventual outcome of inflammatory lesions. Interestingly, mice made deficient for IL-10, by targeted gene disruption, develop bowel inflammation and lesions resembling inflammatory bowel disease, which can be improved by systemic or topical administration of IL-10 (Kuhn et al. 1993). The balance between proinflammatory cytokines such as interleukin-1. interleukin-6, y-interferon and tumor necrosis factor and anti-inflammatory cytokines such as receptor antagonists, IL-10, IL-4, and transforming growth factor beta (TGF^{β}) might ultimately determine the outcome of inflammatory bowel disease (Fiocchi 1993) . By its nature in down regulating pro-inflammatory cytokines, and up regulation of interleukin-1 receptor antagonists, IL-10 may be an effective strategy to treat inflammatory bowel disease. The present treatments for inflammatory bowel disease are empirically designed to reduce inflammation and rely heavily on corticosteroids and immuno-solisolates (5-ASA).

Administration of cytokines orally may offer an alternative to systemic application or topical application and may have local and systemic effects. Interestingly acid inactivation and protease digestion does not appear to inactivate all cytokine effects, in the limited experience to date using an oral approach. Bioactivity may be retained even in the presence of trypsin and chymotrypsin, at least for IL-6, suggesting heavy glycosylation and other factors confer acids stability to cytokines (Rollwagen and Baqar 1996). Furthermore, interferons can suppress collagen induced arthritis in rats when given in high dose orally. Oral cytokines may simplify an approach to delivery and could be useful in modele of infection, HIV, autoimmunity and transplantation rejection.

Human IL10 has also been cloned, and both mouse and human cDNA encode functionally and structurally similar proteins (with 178 amino acids) including a hydrophobic leader sequence and greater than 70% homology in predicted amino acid sequences.

In addition to production by CD4+ TH-2 clones, IL-10 is produced by B cells, activated mast cells, macrophages, monocytes and keratinocytes.There are multiple roles for IL-10 in the regulation of the immune response, including inhibition of T cell, monocyte and macrophage function, as well as inhibition of cytokines particularly those of the TH-1 T cell subset (interferon gamma) and inhibition of B cell proliferation. The inhibition of various cytokines such as interferon gamma. GM-CSF and TNF following lectin or anti-CD3 activation is both transcriptional and translational. There is also inhibitory activity on MHC class 1 antigen expression, which is a protein central to the recognition of antigen presenting cells by T-cell. IL-10 also inhibits the production of hydrogen peroxide and nitric oxide following interferon-gamma activation. Therefore there is a strong basis for the concept that IL-10 naturally occurs to provide negative regulation to immune responses.

Gene constructs were transformed into *N. tabacum* cv. Xanthi and 81 V9-4 tobacco strains (available from Agricuture and Agri-Food Canada, Pest Management Research Center, Delhi Farm. Genetics Section) . 81 V9-4 is a flue-cured tobacco line containing only trace amounts of alkaloids and is an ideal component for tobacco-based molecular farming applications. Gene constructs were built for production of pro and mature forms of a Type II antifreeze protein (AFP) or IL-4, to be accumulated in the cell cytosol and extracelluar space. Several different constructs comprising IL-10 in order to optimize expression of the gene within a non-food crop plant were also examined.

IgG expression in plants lends support to the idea that passage through the endoplasmic reticulum may enhance protein accumulation. In plants bred to simultaneously express mouse IgG light and heavy chain proteins targeted to the extracelluar space, the amount of each protein increased up to 60-fold over that seen when expressed individually into the same compartment (Hiatt et al 1989). Expression of both protein components into the cytosol, however, did not affect their level of accumulation. IgG processing and assembly in lymphocytes occurs through the action of heavy-chain binding proteins present in the endoplasmic reticulum (ER). The observed increase in yield when both proteins were targeted to the extracelluar space has been attributed to an enhanced stability contingent on IgG assembly. Consistent with this hypothesis, active antibody complexes were observed when the proteins were targeted to the extracelluar space but not when targeted to the cytosol.

To further maximize expression levels and transgene protein production the gene encoding the protein of interest, the codon usage within the non-crop plant of interest should be determined. Also, it has been shown that endoplasmic reticulum retention signals can dramatically increase transgene protein levels, for example the KDEL motif (Schouten et al 1996). Replacing any secretory signal sequence with a plant secretory signal will also ensure targeting to the endoplasmic reticulum (Denecke et al 1990).

### Examples

### Examples 1 and 2 are for comparative purpose.

### Gene Construction

To maximize potential AFP production at the translational level, the native 5'-untranslated region of the fish cDNA was replaced with the 5'-untranslated leader region of the tobacco mosaic virus (TMV) (Richards et al 1977 & 1978, Sleat et al 1988). The fish signal peptide sequence was also replaced with that of the tobacco pathogenesis-related protein 1b (PR-1b) (Cornelissen et al 1986, Sijmons et al 1990, Denecke et al 1990). Oligonucleotide cassettes were designed: three oligonucleotides, #1091, (SEQ ID NO:1); # 1092 (SEQ ID NO:4); and # 1309 (SEQ ID NO:2), which would hybridize to generate the complete TMV leader sequence (TMV Cassette: Figure 1A); and five oligonucleotides #4361-#4365 (SEQ ID NOs: 3, 5, 6, 8 and 9, respetively) which in concert with oligonucleotides #1091 and #1092 (SEQ ID NO:1 and 4) would hybridize to generate a linked TMV leader and PR-1b signal peptide DNA sequence (TMV-PR Cassette: Figure 1B). The 5' end of these cassettes corresponded to a *Bam*H I sticky end restriction site. The 3' end of the TMV cassette was blunt and incorporated an additional adenine nucleotide intended to form the first nucleotide of the start codon while the 3' end of the TMV-PR cassette was compatible with *Nde* I-cut DNA but would not be able to re-generate the restriction site following ligation.

To facilitate addition of the TMV and TMV-PR cassettes, sea raven Type II AFP cDNA was site-specifically mutated according to the method described by Kunkel (1985) to incorporate an *Nde* I restriction site at either bp 157 or 207. These mutations also introduced appropriate in-frame methionine start codons at the junctions between sequences encoding pre and pro, or pro and mature portions of the AFP. To build the gene construct for accumulation of AFP into the cytosol, plasmids containing the mutated Type II AFP cDNAs were initially cut with *Nde*I and made blunt-ended with mung bean nuclease. The cDNA fragments were released by cleavage with *Sal* I, were isolated and directionally ligated into a *Bam*H I/*Sal* I-cut pTZ 18 vector together with annealed TMV oligonucleotides #1091, #1092 and #1309. The TMV-proAFP and TMV-mAFP constructs were then cut with *Bam*H I and *Hinc* II, and the isolated AFP gene fragments ligated separately into pMON 893. For gene constructs targeting AFP to the extracelluar space, the mutated Type II AFP cDNAs were initially subcloned from their pTZ 19 vectors into *Hin*d III/*Sal* I-cut pBluescript vectors to position a *Bam*H I restriction site 5' to the AFP coding sequence. The pBluescript-AFP clones were cut with *Bam*H I and *Nde* I restriction enzymes to remove the sea raven signal peptide sequence or signal peptide and pro-region DNA. The plasmid portions of these digests were isolated and ligated with annealed oligonucleotides #1091, #1092, and #4361 through #=136. The gene constructs were then cut with *Xba* I and *Kpn* I, and separately cloned into pCDX-1.

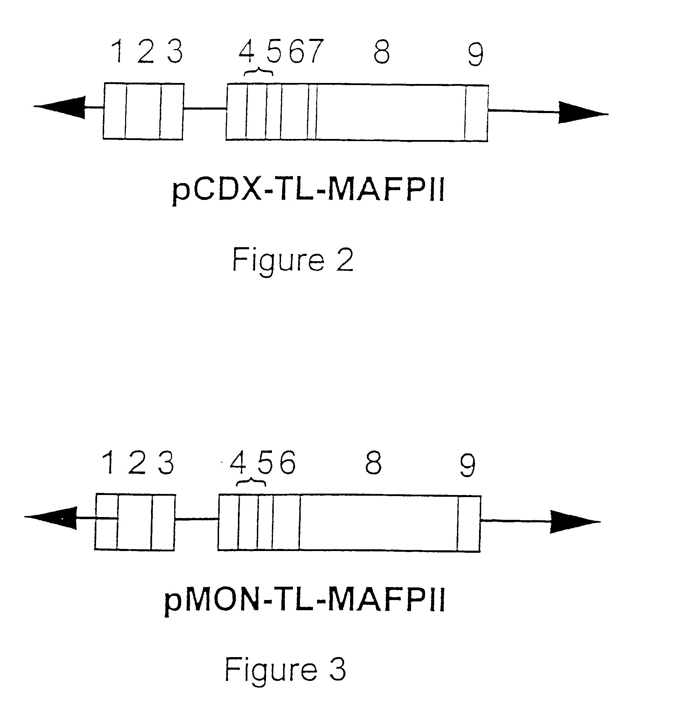

Cloning of the Type II AFP gene constructs into pMON 893 or pCDX-1 oriented the gene cassette into the vector between the double CaMV 35S promoter (Kay et al 1987) and the NOS polyadenylation sequence. AFP encoded by the transgene constructs was identical to the pro and mature AFP forms present in fish with the exception of one additional methionine at the N-terminal end of the proteins. The T-DNA portion of the final constructs are depicted in Figures 2 and 3. Cleavage of the signal peptide components from the expressed AFPs was predicted (von Heijne 1986) to occur immediately prior to the added methionine in both the pro and mature AFP gene constructs so the AFP accumulating in the cytosol and extracelluar space should be identical.

Gene constructs were transformed into *N. tabacum* cv. Xanthi and 81 V9-4 tobacco strains according to the method described by Horsch et al (1988). Attempts to generate plants carrying transgenes for cytosolic accumulation of the AFP were made intermittently over a period of several years. Discs infected with *A. tumefaciens* carrying these gene constructs showed little tendency to form callus and seemed more subject to bacterial infection than comparably treated discs transformed with other transgene constructs. Eventually six transgenic plants were regenerated: one proAFP transgenic in each tobacco strain, three mature AFP transgenics in Xanthi and one mature AFP transgenic in 81 V9-4. By contrast, within six months, eight transgenic plants were regenerated carrying gene constructs for AFP accumulation into the extracellular space: two proAFP transgenics in each strain and four mature AFP transgenics in 81 V-9. Transformed and regenerated plants were initially selected for kanamycin resistance. Transgenic status was subsequently determined by direct PCR screening for the AFP transgene using primers #3339 5'-TATTTTTTACAACAATTACCAACAAC-3' (SEQ ID NO:10) and #4708 5'-CAGCAGTCATCTGCATACAGCAC-3' (SEQ ID NO:11) which hybridize to the TMV leader and at the 3' end of the sea raven AFP coding sequence, respectively. Type II AFP gene constructs were amplified in 30 cycles of denaturation for 1 min at 95 °C, annealing for 1 min at 55°C, and elongation for 2 min at 72°C. This generated amplification products of 440 and 388 bp from the gene constructs for cytosolic accumulation of the pro and mature AFPs, and 532 and 480 bp fragments from gene constructs designed for accumulation of the same AFPs into the extracellular space.

### Analysis of Transgene Expression

Plants were removed from the growth chamber and maintained at room temperature under a grow light for a minimum 48 h prior to RNA or protein extraction. RNA was prepared by selective precipitation according to the method of Palmiter (1974). Total RNA (40 µg) was analysed by Northern blots according to the method of Lehrach et al (1977) and probed with [α-³²P]-labelled sea raven AFP cDNA sequence.

Total soluble protein extracts were prepared from three to four leaves taken from the upper half of a healthy plant according to the method described by Gensenheimer (1990). Extracts were dialysed at 4°C against 0.1%, 0.01% and 0.001% ascorbic acid in SpectraPor^{®} #3 dialysis tubing (MWCO 3.5 kDa) and centrifuged for 15 min at 12.000xg to remove precipitate formed during dialysis prior to lyophilization. Samples were resuspended in Millipore^{®}-filtered water and their protein concentrations determined by Bradford assay (16) relative to a BSA standard.

To extract protein present in the appoplast by vacuum infiltration (Sijmones et al 1990), leaves were cut longitudinally into 4 cm x 0.8 cm strips, and rolled lengthwise in 5 cm x 1.8 cm strips of Parafilm^{®} so that the bottom of the leaf was exposed to view. The leaf strip was exposed to vacuum derived from a water aspirator twice for 2 min or until the exposed leaf surface was dark green. Each treated leaf strip yielded 10 ± 2.5 ul of extract. Larger volume samples were too dilute for use and were discarded. Samples which contained green pellets were also discarded to reduce the possibility of cytosolic protein contamination. All remaining extracts from a given plant were pooled and the amount of protein present determined by Bradford (1976) protein assay relative to a BSA standard. Extract concentrations were generally between 0.1-0.2 µg/µl.

### Western Analysis

Lyophilized protein was resuspended directly in loading buffer (60 mM Tris-HCl, pH 6.8/10% glycerol/5% β-mercaptoethanol/2% (w/v) SDS/ 1.3 x 10⁻³% (w/v) bromophenol blue), and were electrophoresed through a 17% polyacrylamide/0.1 M sodium phosphate, pH 6.8/4 M Urea/0.1% SDS gel using 0.1 M sodium phosphate, pH 6.8/0.1% SDS running buffer. Electrophoresis was conducted at 50 V for 3-4 h or until the 6 kDa pre-stained marker was at the bottom of the gel. Western blots were prepared according to the method described by Burnette et al (1981) and probed with polyclonal antibody to the mature sea raven AFP. A chemiluminescence detection kit (Amersham) for probing of Western blots was used to detect bound first antibody. All reactions were carried out according to the manufacturer's directions.

### Example 1: AFP Gene Expression

### Protein Accumulation

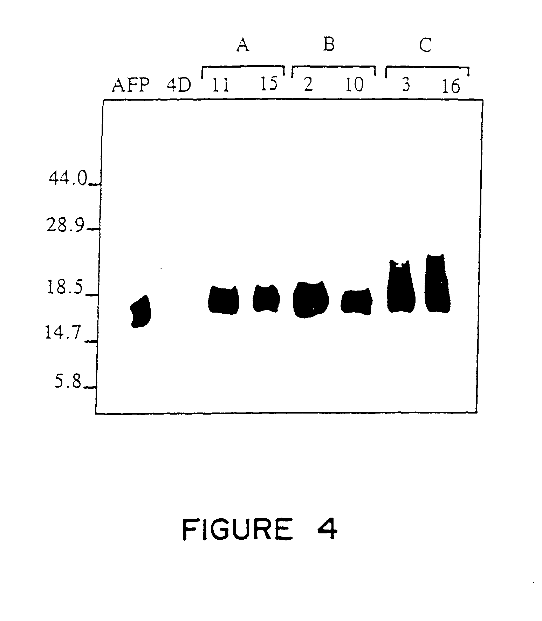

Western blot analysis of total soluble protein extracted from plants carrying genes for cytosolic AFP expression did not find evidence of either pro or mature AFP accumulation even when up to 80 µg of protein extract were analysed. In contrast, a protein which co-migrated with mature AFP from sea raven and cross-reacted with antibody to Type II AFP was detected in as little as 2.5 µg total soluble protein extract of plants in which the AFP was targeted to the extracellular space (Figure 4). This protein was unique to the transgenic plants and was absent in extracts of a wild-type plant and a plant, pII3a-#9, which had been transformed and regenerated but determined to be non-transgenic by PCR. Insufficient resolution was obtained with these extracts to differentiate size differences between the AFPs produced by the plants carrying transgenes for production of pro or mature AFPs. This was probably due to the viscosity of the total soluble protein extracts which often caused distortion of samples during the running of gels. For this reason, positive control Type II AFP was mixed with total soluble protein extract from a lab-grown wild-type plant to generate a suitable size standard. Wild-type protein extract was not mixed with the molecular weight markers, so the size of plant-produced AFP could not accurately estimated from these extracts. AFP accumulation varied between plants and appeared slightly higher in plants II4c²-#1 and 10 which carried genes for mature AFP production. Based on Western blot comparison with a known amount of Type II AFP, the amount of AFP produced by the plants has been estimated to be approximately 0.5-1% of the total soluble protein.

### mRNA Transcription from Transgenes for Cytosolic AFP Accumulation

RNA extracts of plants transgenic with gene constructs for cytosolic AFP accumulation were analysed by Northern blot to determine if the AFP transgene was transcribed. Two plants, Xanthi TmSR # 1 and 8 1 V9-4 TmSR #1, carrying transgenes for AFP accumulation into the cell cytosol were found to produce a 0.96 kb RNA transcript which hybridized with the AFP cDNA used as a probe: on longer exposure of the blots, the same RNA transcript was also seen in extract from Xanthi TmSR #2 and slightly larger one of approximately 1.02 kb was detected in extract from 81V9-4 TpSR #3. Transcripts from pro and mature AFP transgene constructs were expected to differ by 51 nucleotides. Both plant-produced AFP gene transcripts were both considerably larger than a PCR-generated control sample estimated to be 0.36 kb and representing the size of the mature AFP transgene between the TMV leader and the 3' end of the coding sequence. This was consistent with the size difference expected based on the additional coding sequence and 3'-untranslated region (155 nt total) present in the transgene construct, and with the use of the NOS polyadenylation sequence to obtain polyadenylated transcripts. Both transcripts were smaller than an equivalent gene transcript of 1.06 kb produced in a plant carrying the transgene for targeted mature AFP accumulation into the extracellular space. This size difference was consistent with the addition of the 90 nt signal peptide coding sequence to the transgene construct for secretion of the AFP. Based on EtBr staining, more RNA was loaded onto the gel from the plant producing AFP for accumulation into the extracellular space compared to the amount of RNA from the plants targeting the AFP to the cytosol. No comparison could be made regarding the relative amounts of AFP mRNA transcribed from these constructs. However, similar loadings were achieved between the plants targeting the AFP to the cytosol. The difference in amount of transcript observed, therefore, suggests that this transgene construct was being expressed at variable levels in the different plants.

### AFP Present in the Apoplast

Vacuum infiltration extracts were prepared from AFP-producing plants to determine if the expressed AFP was present in the extracellular space. These extracts were not subject to the viscosity and sample distortion associated with total soluble protein extracts, so were also considered more suitable to estimate the sizes of the plant AFPs. Western blot analysis confirmed the presence of AFP. AFP was detected in plants pII3a-#7 and II4c²-#1 which carried mature and proAFP transgenes respectively, as a diffuse band of approximately 14.7 kDa which migrated at a similar rate to mature AFP isolated from fish sera. A small mobility difference was observed between the AFPs produced by the two plants, however, proAFP isolated from fish sera was distinctly larger than either plant-produced AFP.

Having established the presence of AFP in vacuum infiltration extracts of those plants carrying transgenes for extracellular AFP accumulation, the extracts were concentrated ten-fold and tested for thermal hysteresis activity and effects on ice crystal morphology using the nanolitre osmometer. Extracts from plants expressing gene constructs for either the pro or mature AFP both showed evidence of AFP activity. Ice crystals grown in these plant extracts were seen as the characteristic eye-shaped bipyramids produced in the presence of native Type II AFP. Thermal hysteresis activity was measured and based on comparison with a standard activity curve the amount of active AFP present in the extracellular space was estimated as 2% of the total protein present in the compartment.

RNA analysis of the transgenic plants has shown that they are able to transcribe the integrated transgene constructs, and that the size of the plant AFP mRNA from both construct types is consistent with that expected for a full-length, polyadenylated transcript. mRNA produced from the transgene for AFP accumulation into the extracellular space was obviously translatable.

The presence of plant-produced AFPs in vacuum infiltration extracts indicates that the plant recognized the PR-1b signal peptide component and correctly targeted the attached AFPs to the extracellular space. The predicted size of the AFPs for export to the extracellular space is 18.7 or 17 kDa with the signal peptide component attached and 15.8 or 14.1 kDa without the signal peptide for the pro and mature AFPs, respectively. Western blot analysis of the plant vacuum infiltration extracts showed that the AFPs produced from the proAFP and mature AFP transgene constructs and present in the extracellular space both co-migrated with mature Type II AFP and were approximately 14.7 kDa. This suggests that not only is the plant capable of cleaving the signal peptide from the expressed AFP but that it can remove most or all of the pro-region of the AFP.

Thereore, this example demonstrates that a low alkaloid plant is capable of expressing an imminologically detectable protein of interest that retains its native activity.

### Example 2: Interleukin-4 expression

Full length murine IL-4 cDNA is 585 base pairs long (Lee et al 1988) and codes for a 140 amino acid preprotein with a 40 amino acid secretory signal (Otsuka et al 1987). This gene sequence encoding the 40 amino acid secretory signal was replaced with a plant secretory signal obtained from the tobacco PR1-b secretory signal (Denecke et al 1990) to ensure targeting to the endoplasmic reticulum. The resultant vector was introduced into low-nicotine tobacco plants using the methods described above.

In order to determine the biological activity of plant recombinant IL-4, *in vitro,* the stimulation of growth of murine IL-4 dependent CT.4S cell line (Rapoport et al 1993) is determined. Appropriate dilutions of purified plant recombinant IL-4 is added to cultures containing 5x10³ CT.4S cells in flat bottom 96-well plates in a final volume of 100 ml for 48 hr. CT.4S cell proliferation is assessed by addition of 1 mCi/well of [³H]thymidine 18 hr before termination of culture, and [³H] thymidine incorporation is determined by liquid scintillation counting.

The immunoreactivity of plant recombinant IL-4 is determined by solid phase sandwich ELISA (Abrams et al 1992). Briefly, the BVD4-1D11 anti-IL-4 mAB is used as a capture antibody and is paired with the biotinylated BVD6-24G2 antiIL-4 mAB as the detecting antibody. Mouse rIL-4 (Pharmingen) is used a standard.

In vivo activity of plant produced IL-4 is determined in NOD mice. NOD and control strain (insulitis- and diabetes-free) female mice (20 mice/group) are fed either transgenic low nicotine tobacco leaves expressing recombinantly produced IL-4. At various times during the 4 week treatment, circulating serum levels of plant recombinant IL-4-fed and un-fed NOD and control mice are analyzed by ELISA as described above. Both NOD and control mice are monitored for any potential toxic effects arising from the plant recombinant IL-4 feeding, and their serum concentrations of IgE and IgG quantified by ELISA. Increase in serum IgE levels indicate that recipient mice are more susceptible to allergic responses.

Feeding of plant recombinant IL-4 to protect against the onset of insulitis and/or Type 1 diabetes in NOD mice is also determined. Neonatal female NOD mice (2-3 weeks of age) fed or not fed with plant recombinant IL-4 are maintained in a specific pathogen free animal facility. Mice are monitored weekly for their blood glucose levels (BGL), and mice that are hyperglycemic (i.e. BGL > 11.1 mmol/L) for two consecutive weeks are diagnosed as Type 1 diabetic. The onset of insulitis is monitored by immunohistochemical analysis of pancreatic tissue by sacrificing mice at various times (2 weeks to 2 months) after plant recombinant IL-4-feeding is initiated.

### Example 3: Interleukin-10 (IL-10) expression

Full length mRNA encoding human IL-10 (hIL-10) is 1601 nucleotides long, with a short 5' UTR of 30 nucleotides (nt) and a long 3' UTR of 1037 nt. The hIL-10 coding sequence is 534 base pairs long and codes for a 178 amino acid preprotein with a 18 amino acid secretory signal (Vieira et al., 1991). The full length human IL10 cDNA is isolated from polyA RNA by RT-PCR and fully sequenced to ensure fidelity. In order to maximize expression levels and transgene protein production, the IL 10 cDNA is examined for codon usage patterns and optimized as necessary (Perlak et al. 1991).

Since ER retention signals can increase the concentration of disulfide bonded transgene proteins, therefore the KDEL ER retention motif may be added to the IL-10 cDNA using site directed mutagenesis. For comparative purposes an identical synthetic gene, without the KDEL signal, is prepared and used in the transformation studies (SEQ ID NO:12).

The expression system adopts a duplicated 35S enhancer-promoter plus AMV leader sequence (Kay et al. 1987; Jobbing and Gehrke 1987). However other constitutive promoters may also be employed, for example. Agriculture and Agrifood Canadas T1276 constitutive promoter from tobacco (see copending application US application serial No.08/593,121). The IL-10 gene is cloned into a T-DNA vector between the T-DNA border sequences using the Bin19 derived plant expression vector pCamTerX. The synthetic IL-10 gene is transformed to tobacco using *Agrobacterium* mediated transformation (Horsch et al. 1989). The *Agrobacterium* strain used is EHA 101 carrying the disarmed Ti plasmid pEHA 104. The plant selectable marker is neomycin phosphotransferase.

To maximize transgene expression and ensure stable field performance a large number of primary transformants need to be generated, selected for expression levels, screened for copy number, and single-copy high-expressing lines evaluated for field performance. Standard methods (e.g. Northem and Western blotting) are used to evaluate expression levels (Sambrook et al. 1989). ELISA is used to quantify transgene protein yields.

A sequence containing the hIL-10 open reading frame and a truncated 3' UTR (nt 4-732) is amplified by PCR using oligonuleotides hIL-10-5'-BanxHI and hIL-10-3'-EcoRI (Figure 5(B): transformed plants comprising this contruct are denoted "E" in Figure 7). The 5' oligonucleotide is designed with a BamHI site at the 5' end, and the 3' oligo is designed with an EcoRI site at the 5' end. These restriction sites enable the easy directional cloning of the construct in the phagemid pBluescript (Stratagene) and in the Bin 19-derived binary vector pCaMter X (Frisch et al., 1995). To determine the effect of the 3' UTR on expression of hIL-10 in plants, the complete cDNA (4-1601; Figure 5(A); transformed plants comprising this contruct are denoted "F" in Figure 7) is also cloned into pBluescript and pCaMter X.

To maximize protein accumulation in the plant cell, the endoplasmic reticulum (ER) retention signal. KDEL (Schouten et al., 1996), is added to the 3' end of hIL-10 coding sequence; transformed plants comprising this contruct are denoted "G" in Figure 7). A His tag is also added before the KDEL signal to facilitate purification of the transgenic protein (see Figure 5 (C), and SEQ ID NO:12, nucleotides 544-630). A thrombin recognition sequence is inserted immediately preceding the His tag that allows the cleavage of the His tag and the KDEL peptides. This third construct encodes a preprotein of 197 amino acids. A single oligonucleotide is used to fuse these sequences to the end of hIL-10 reading frame (see Figures 5 and 6). This oligonucleotide is used in conjunction with oligonucleotide hIL-10-5'-BamHI in a PCR to generate the fragment which was then cloned both in pBluescript and in pCaMter X.

Cloning of the IL-10 gene constructs into pCaMter X orientes the gene between the double CaMS double 35S promoter (Kay et al., 1987) and the NOS (Nopaline synthase) polyadenylation sequence (Frisch et al., 1995). The plant recombinant IL-10 (prIL-10) encoded by the native transgene constructs was identical to the native human IL-10. After purification and thrombin cleavage, the prIL-10 encoded by the tagged construct differs in only its two terminal amino acids from the native hIL-10..

Gene constructs are transformed into *N*. *tabacum* cv. 81 V9-4 low alkaloid tobacco strains according to the method described by Miki et al. (1998). Transformed and regenerated plants are initially selected for kanamycin resistance. Regenerated plants are analyzed for expression of the IL10 protein by solid phase sandwich Enzyme-Linked ImmunoSorbent Assay (ELISA). Briefly, the JES3-9D7 anti-IL-10 mAB (PharMingen) is used as a capture antibody and is paired with the biotinylated JES3-12G8 anti-IL-10 mAB (PharMingen) as the detecting antibody. Recombinant human IL-10 (PharMingen) is used as standard.

Total soluble protein extracts are prepared from 1 gram of young leaf tissue by homogenizing the tissue in 1 ml of a buffer containing phosphate buffered saline-Tween (PBS-T), 2% polyvinylpolypyrrolidone (PVPP), 1mM ethylenediaminetetraacetic acid (EDTA), 1 mM phenylmethyl sulfonyl fluoride (PMSF) and 1 µ/ml Leupeptin. The homogenate is then collected in a tube and spun at 10,000 g for 15 min in a refrigerated centrifuge. The supernatant is collected and used directly in the ELISA. The results are presented in Figure 7.

Eighteen transgenic plants are regenerated from the native construct containing only a short stretch of 3' UTR (called E6 and E 15 in Figure 7), and nineteen plants are regenerated from the native construct containing the entire 3' UTR (called F10 in Figure 7). All of those and the first two plants regenerated from the construct containing the His tag and KDEL ER retention signal (called G7 in Figure 7) are tested for presence of IL-10 protein by ELISA.

A variable level of plant recombinant IL-10 (prIL-10) is observed in the regenerated plants. There were no marked differences between plants transformed with the full-length hIL-10 cDNA and those transformed with 3' truncated cDNA. However, the highest level of prIL-10 is obtained in one of the two plants transformed with the construct containing the ER retention signal G7-2.

Following the detection of IL-10 transcripts and protein in the transgenic plants crude protein extracts containing prlL-10 the demonstration of bioactivity of human IL-10 is determined *in vitro* by its effect on T cell proliferation in response to lectins (PHA) as well as in standard mixed lymphocyte responses (MLR). Plant extract or purified IL-10 from plant extract is added to media containing human lymphocytes, at a concentration ranging from 1-100 ngm/ml. Proliferation is determined in PHA cultures and mixed lymphocyte reaction cultures at days 3-6 by standard ³H- thymidine incorporation proliferation assays.

Surface expression of MHC class II proteins is determined on human lymphocytes activated by either PHA or ConA in the presence of increasing concentrations of IL-10. And compared to control plant extracts.

Peripheral blood lymphocytes are stimulated with LPS (1-10 µ/ml) for 24-48 hours, and levels of cytokines determined by ELISA or bioassay. Inhibition is determined as well, at the RNA level, by Northern blot analysis.

IL-10 is beneficial in prevention of lethal endotoxemia. Following appropriate approval the effect of IL-10 purified from plant extract on endotoxic injury in BALB/c mice is tested. *E.coli* is administered interperitoneally or intravenously in the presence of various amounts of recombinant munne IL 10. Plant extract is given to mice orally in addition to purified IL-10 from plants given intravenously. Outcomes are determined by survival of mice, which is essentially zero percent in untreated mice. Appropriate controls include PBS treated mice as well as mice given standard animal chow.

IL-10 deficient mice develop bowel inflammation and lesions resembling inflammatory bowel disease, which is improved by systemic IL-10. To test whether oral plant IL-10 can prevent bowel disease in an IL-10 deficient mouse model of IBD, transgenic plant material is administered to IL-10 null mice daily from age 4 weeks and mice are assessed for the presence of bowel lesions from age 8 to 30 weeks by weight gain and histology of tissue.

### Example 4: Alkaloid levels in leaf tissue of the non-food bioreactor

In order to establish concentrations of tobacco alkaloids in normal and non-food bioreactor plants a field trial was conducted during the summer of 1997. Transplants of Delgold, a tobacco cultivar with normal alkaloid concentrations and 81 V-9 the non-food bioreactor genotype used for this present invention were started in mid April in a cold frame greenhouse located at the Delhi Research Station in Ontario Canada. The two cultivars were transplanted into a 1.2 x 2 m plots at a density of approximately 133,000 plants per hectare. The trial employed a randomized complete design with four replications and was fertilized with 60 kg nitrogen per hectare. The trial was harvested 40 days after planting, the tissue was roughly chopped, quick frozen in liquid nitrogen and held at -70 °C until ready for analysis. The tissue was then lyophillized and tobacco alkaloid concentrations in the tissue determined by gas chromatography. For this analysis approximately 1 gram of lyophilized tobacco tissue was ground to pass a 20 mesh screen and was then extracted with dichloromethane and aqueous sodium hydroxide by shaking for 10 min on a wrist action shaker. The dichloromethane layer was filtered and collected for alkaloid analysis. The internal standard method was used with anethole being that standard. All gas chromatographic analyses were performed on an Hewlett Packard 5890 series II using a DB5 60m x.25mm i.d. column with splitless injection and a 2 µl sample volume. Total tobacco alkaloid concentrations were found to be 35 fold lower in the non-food bioreactor than the normal tobacco cultivar, nicotine concntration had a similar pattern to the total and accounted for between 92 and 99 % of the total (Table 1). The levels of anabasine were similar in the two lines and those of anatabine higher in the non-food bioreactor than Delgold.

**Table 1.**

| **Concentration (mg/g) of the various tobacco alkaloids found in the leaves of normal tobacco and in the non-food bioreactor.** | | | | | | | |
|---|---|---|---|---|---|---|---|
| Cultivar | Nicotine | Myosmine | Anabasine | Anatabine | Total | % of total nicotine | % of total alkaloid |
| | ---------------------------------------------------- mg/g -------------------------- | | | | | | |
| Delgold | 7.892a* | 0.000a | 0.013a | 0.000a | 7.923a | 100 | 100 |
| 81 V-9 | 0.209b | 0.000a | 0.018a | 0.020b | 0.227b | 2.6 | 0.28 |

| | | | | | | | |
|---|---|---|---|---|---|---|---|
| *means followed by a different letter are significantly from different from each other at P=0.05 | | | | | | | |

The present invention has been described with regard to preferred embodiments. However, it will be obvious to persons skilled in the art that a number of variations and modifications can be made without departing from the scope of the invention as described in the following claims.

### References

Abrams. J.S., M.G. Roncarolo, H. Yasel, U. Anderson, G. Gleich. J. Silver (1992) Strategies of anti-cytokine monoclonal antibody development. Immunol. Rev. 127, 5-24.
Bradford M.M. (1976) A rapid and sensitive method for the quantitation of microgram quantities of protein utilizing the principle of protein dye binding. Anal. Biochem. 72, 248-254.
Burnette W.H. (1981) Western blotting: electrophoretic transfer of proteins from SDS-polyacrylamide gels to unmodified nitrocellulose and radio-graphic detections with antibody and radioiodinated protein A. Anal. Biochem. 112, 195-203.
Chaplin J.F. (1977) Breeding for varying levels of nicotine in tobacco. In: Proc Amer Chem Soc, 173rd meeting. New Orleans.
Cornelissen B.J.C, R.A.M Hooft van Huijsduijnen, L.D.Van Loon & J.F.Bol (1986) Molecular characterization of messenger mRNAs for "pathogenesis-related" proteins la, 1b and 1c, induced by TMV infection of tobacco. EMBO J. 5, 37-40.
Denecke J., J.Botterman & R.Deblaere (1990) Protein secretion in plant cells can occur via a default pathway. Plant Cell 2, 51-59.
Fourney R.M., J.Miyakoshi, R.S.Day III & M.C.Paterson (1988) Northern blotting: efficient RNA staining and transfer. Focus 10:1, 5-7.
Fiocchi C. (1993) Cytokines and animal models: a combined path to inflammatory bowel disease pathogenesis. Gastroenterology 104:1202-1219.
Frisch, D. A., Harris-Haller, L.W., Yokubaitis, N.T., Thomas, T.L., Hardin, S.H., and Hall, T.C. (1995). Complete sequence of the binary vector Bin 19. Plant Mol. Biol. 27, 405-409.
Gengenheimer P (1990) Preparation of Extracts from Plants. Methods of Enzymology 182, 184-185.
Haq T.A., H.S. Mason, J.D. Clements, C.J. Artzen (1995) Oral immunization with a recombinant bacterial antigen produced in plants. Science 268, 714-716.
Hiatt A., R.Cafferkey & K.Bowdish (1989) Production of antibodies in transgenic plants. Nature 342. 76-78.
Horsch R.B., J.Fry, N.Hofmann, J.Neidermeyer, S.G.Rogers & R.T.Fraley, (1988) Leaf disc transformation. Plant Molecular Biology Manual A5. 1-9. Ed. S.B.Gelvin. R.A.Schilperoort, D.P.S.Verma. Kluwer Academic Publishers. Dordrecht/Boston/London.
Kay R., A. Chan, M.Daly & J.McPherson (1987) Duplication of CaMV 35S promoter sequences creates a strong enhancer for plant genes. Science 236, 1299-1302.
Kuhn R. LohlerJ, Rennick D, Rajewsky K, Muller W (1993) Interleukin-10 deficient mice develop chronic enterocolitis. Cell 75:263-274.
Kunkel T.A. (1985) Rapid and efficient site-specific mutagenesis without phenotypic selection. Proc. Natl. Acad. Sci. USA 82, 488-492.
Lehrach H., D.Diamond, J.M. Wozney & H. Boedtker. (1977) RNA molecular weight determinations by gel electrophoresis underdenaturing conditions: a critical reexamination. Biochemistry 16,4743.
Ma J.K.C., M. Hein (1995) Immunotherapeutic potential of antibodies produced in plants. TibTech 13, 522-527.
Mason H.S., J.M. Ball, J-J. Shi, X. Jiang, M.K. Estes, C.J. Arntzen (1996) Expression of Norwalk virus capsid protein in transgenic tobacco and potato and its oral immunogenicity in mice. Proc. Nat. Acad. Sci. 93, 5335-5340.
Miki, B.L.A., McHugh, S.G., Labbe, H., Ouellet, T., Tolman, J.H., and Brandle, J.E. (1998) Transgenic tobacco: gene expression and applications. Biotechnology in Agriculture and Forestry (in press)
Montanari L., P. Fantozzi, S. Pedone (1993) Tobacco fraction 1 (F1P utilization of oral feeding and enteral feeding of patients. 1 Heavy Metal evaluation. Food SAci. Tech. 26, 259-263:
Otsuka T., D. Vallaret, T. Yokota, Y. Talcebe, F. Lee, N. Arai, K. Arai (1987) Structural analysis of the mouse chromosomal gene encoding interleukin-4 which expresses B cell, T cell and mast cell stimulating activities. Nucl. Acid Res. 15, 333-334
Palmiter R.D. (1974) Magnesium precipitation of ribonucleoprotein complexes: Expedient techniques for the isolation of undegraded polysomes and messenger ribonucleic acid. Biochemistry 13, 3606-3615.
Perlak FJ, Fuchs RL, Dean DA, McPherson SL, Fischoff DA (1991) Modification of the coding sequence enhances plant expression of insect control protein genes. Proc Natl Acad Sci 88:3324-3328.
Rapoport M.J., D. Zipris. A.H. Lazarus. A. Jaramillo, D.V. Serreze. E.H. Leiter. P. Cyopick, J.S. Danska, T.L. Delovitch (1993) IL-4 reverses thymic T cell proliferative unresponsiveness and prevents diabetes in NOD mice. J. Exp. Med. 178. 87-99.
Richards K., H.Guilley, G.Jonard & G.Keith (1977) Leader sequence of 71 nucleotides devoid of G in tobacco mosaic virus RNA. Nature 267, 548-550.
Richards K., H.Guilley, G.Jonard & L.Hirth (1978) Nucleotide sequence at the 5' extremity of tobacco mosaic virus RNA. Eur. J. Biochem. 84, 513-519.
Schouten A et al. (1966) The C-terminal KDEL sequence increases the expression level of a single-chain antibody designed to be targeted to both the cytosol and the secretory pathway in transgenic tobacco. Plant Molec. Biol. 30, 781-793.
Sleat D.E.. R.Jull, P.C.Turner & T.M.A. Wilson (1988) On the mechanism of translational enhancement by the 5'-leader sequence of tobacco mosaic virus RNA. Eur. J. Biochem. 175, 75-86.
Sijmons P.C., B.M.M.Dekker, B.Schrammeijer, T.C.Verwoerd, P.J.M.van den Elzen & A.Hoekema (1990) Production of correctly processed human serum albumin in transgenic plants. Bio/Technology 8, 217-221.
Vieira, P., de Waal-Maleyfyt, R., Dang, M.N., Johnson, K.E., Kastelein, R., Fiorentino, D.F., de Vries, J.E., Roncarolo, M.G., Mosmann, T.R., and Moore, K.W. (1991) Isolation and expression of human cytokine synthesis inhibitory factor cDNA clones: homology to Epstein-Barr virus open reading frame BCRF1. Proc. Natl. Acad. Sci. U.S.A. 88, 1172-1176.
von Heijne G. (1986) A new method for predicting signal cleavage sites. Nucl. Acids Res. 14,4683-4690.
Woodleif W.G., J.F. Chaplin, C.R. Campbell, D.W. DeJong (1981) Effect of variety and harvest treatments on protein yield of close grown tobacco Tobacco Sci. 25, 83-86.

## Claims

1. A method for the preparation of IL-10 for oral administration, comprising;
i) transforming a non-food crop plant, **characterized** as being non-toxic, non addictive and palatable, with a vector containing a nucleic acid encoding human IL-10 operatively linked with 5'and 3' regulatory regions;
ii) selecting non-food crop plants that express human IL-10 to obtain transformed non-food crop plants;
iii) growing and harvesting said transformed non-food crop plants; and
iv) obtaining plant tissue comprising human IL-10 from said transformed non-food crop plants.

2. The method according to claim 1, wherein said nucleic acid is optimized for codon usage.

3. The method of claim 1 or claim 2 wherein said non-food plant is **characterized in** having a low alkaloid level.

4. The method of claim 3 wherein said non-food crop plant is a tobacco plant **characterized** as having a low nicotine level.

5. The method of claim 4 wherein, in said step of transforming, said 5' regulatory region is selected from the group consisting of ubiquitous and tissue specific promoters.

6. The method of claim 5 wherein, in said step of transforming, said nucleic acid includes an endoplasmic reticulum retention motif.

7. The method of claim 6 wherein said nucleic acid includes a plant signal sequence.

8. The method of claim 1 or claim 2, wherein said plant tissue comprising IL-10 obtained in said step of obtaining (step iv) is for oral administration to an animal.

9. A method for the preparation of IL-10 comprising extracting IL-10 from said transformed non-food crop plants of claim 1 or claim 2.

10. A protein composition prepared by the method of claim 1 or claim 2 comprising IL-10 and plant components.

11. The protein composition of claim 10 wherein the non-food crop is tobacco.

12. The protein composition of claim 10 wherein the plant tissue is leaf.

13. A protein composition prepared by the method of claim 9 comprising IL-10 and soluble plant components.

14. The method of claim 1 wherein, in said step of transforming, said vector also comprises sequences that enhance expression of said IL-10.

15. The method of claim 1 wherein, in said step of transforming, said vector encodes an affinity tag.

16. The method of claim 1 wherein said vector also encodes a proteolytic cleavage site.

17. The method of claim 1, wherein said step of obtaining comprises extracting IL-10 from said transformed plant to produce a crude extract.

18. The method of claim 17, wherein said step of obtaining comprises extracting IL-10 from said transformed plant and partially purifying said IL-10.

19. The method of claim 4, wherein said tobacco plant comprises from 0.2% to 20% of the total alkaloid of Delgold.

20. Use of the protein composition of claim 10 or claim 13 in the preparation of a medicament for treatment of a medical ailment in a patient, wherein the medical ailment is Crohn's disease, collagen induced arthritis, diabetes, conditions related to blood clotting, medical conditions requiring the stimulation of progenitor cells to monocytes/macrophages, viral infections or regulation of immune responses.

## Patentansprüche

1. Verfahren zur Herstellung von IL-10 für die orale Verabreichung umfassend:
i) Transformierung einer Nicht-Lebensmittel Kulturpflanze, **dadurch gekennzeichnet, dass** sie ungiftig, nicht abhängig machend und genießbar ist, mit einem Vektor enthaltend eine Nukleinsäure, codierend für das humane IL-10 und operativ mit 5'- und 3'-regulatorischen Regionen verbunden;
ii) Auswählen der Nicht-Lebensmittel Kulturpflanzen, die das humane IL-10 exprimieren, um transformierte Nicht-Lebensmittel Kulturpflanzen zu erhalten;
iii) Heranziehen und Ernten der transformierten Nicht-Lebensmittel Kulturpflanzen; und
iv) Erhalten von Pflanzengewebe von den transformierten Nicht-Lebensmittel Kulturpflanzen, die das humane IL-10 umfassen.

2. Verfahren nach Anspruch 1, wobei die Nukleinsäure hinsichtlich der Verwendung der Codons optimiert ist.

3. Verfahren nach Anspruch 1 oder Anspruch 2, wobei die Nicht-Lebensmittel Kulturpflanze **dadurch gekennzeichnet ist, dass** sie ein niedriges Alkaloidniveau aufweist.

4. Verfahren nach Anspruch 3, wobei die Nicht-Lebensmittel Kulturpflanze eine Tabakpflanze ist, die **dadurch gekennzeichnet ist, dass** sie ein niedriges Nikotinniveau aufweist.

5. Verfahren nach Anspruch 4, wobei in dem Transformationsschritt die 5`-regulatorische Region ausgewählt ist aus der Gruppe bestehend aus ubiquitär und gewebsspezifischen Promotoren.

6. Verfahren nach Anspruch 5, wobei in dem Transformationsschritt die Nukleinsäure ein Zurückbehaltungsmotiv für das endoplasmatische Retikulum einschließt.

7. Verfahren nach Anspruch 6, wobei die Nukleinsäure eine Pflanzen-Signalsequenz einschließt.

8. Verfahren nach Anspruch 1 oder Anspruch 2, wobei das Pflanzengewebe, das IL-10 umfasst und im Schritt des Erhaltens (Schritt iv) erhalten wurde für die orale Verabreichung an ein Tier bestimmt ist.

9. Verfahren zur Herstellung von IL-10 umfassend Extrahieren von IL-10 aus den transformierten Nicht-Lebensmittel Kulturpflanzen gemäß Anspruch 1 oder Anspruch 2.

10. Proteinzusammensetzung, die nach dem Verfahren nach Anspruch 1 oder Anspruch 2 hergestellt wurde und die IL-10 und Pflanzenbestandteile umfasst.

11. Proteinzusammensetzung nach Anspruch 10, wobei die Nicht-Lebensmittel Kulturpflanze Tabak ist.

12. Proteinzusammensetzung nach Anspruch 10, wobei das Pflanzengewebe aus Blättern besteht.

13. Proteinzusammensetzung hergestellt gemäß des Verfahrens nach Anspruch 9 umfassend IL-10 und lösliche Pflanzenbestandteile.

14. Verfahren nach Anspruch 1, wobei in dem Transformationsschritt der Vektor auch Sequenzen umfasst, die die Expression des IL-10 verstärken.

15. Verfahren nach Anspruch 1, wobei in dem Transformationsschritt der Vektor für ein Affinitäts-Tag codiert.

16. Verfahren nach Anspruch 1, wobei der Vektor auch für eine proteolytische Spaltstelle codiert.

17. Verfahren nach Anspruch 1, wobei der Schritt des Erhaltens die Extraktion von IL-10 von den transformierten Pflanzen umfasst, um ein krudes Extrakt zu erzeugen.

18. Verfahren nach Anspruch 17, wobei der Schritt des Erhaltens die Extraktion von IL-10 aus den transformierten Pflanzen und eine teilweise Aufreinigung des IL-10 umfasst.

19. Verfahren nach Anspruch 4, wobei die Tabakpflanze von 0,2% bis zu 20% des gesamten Alkaloids von Delgold umfasst.

20. Verwendung der Proteinzusammensetzung nach Anspruch 10 oder Anspruch 13 für die Herstellung eines Medikaments zur Behandlung eines medizinischen Leidens eines Patienten, wobei das medizinische Leiden Morbus Crohn, Kollagen-induzierte Arthritis, Diabetes, mit der Blutgerinnung assoziierte Leiden, medizinische Leiden, die die Stimulation von Vorläuferzellen von Monozyten/Makrophagen erfordern, virale Infektionen oder die Regulation von Immunantworten ist/sind.

## Revendications

1. Procédé de préparation d'IL-10 pour l'administration orale comprenant :
i) transformer une plante cultivée non alimentaire, **caractérisée en ce qu'**elle est non toxique, n'entraîne pas d'accoutumance et est de goût agréable, avec un vecteur contenant un acide nucléique codant IL-10 humaine lié de manière fonctionnelle à des régions régulatrices en 5' et 3' ;
ii) sélectionner des plantes cultivées non alimentaires qui expriment IL-10 humaine pour obtenir des plantes cultivées non alimentaires transformées ;
iii) cultiver et récolter lesdites plantes cultivées non alimentaires transformées ; et
iv) obtenir un tissu végétal comprenant IL-10 humaine à partir desdites plantes cultivées non alimentaires transformées.

2. Procédé selon la revendication 1 où ledit acide nucléique est optimisé pour l'usage des codons.

3. Procédé selon la revendication 1 ou la revendication 2 où ladite plante non alimentaire est **caractérisée en ce qu'**elle a un bas niveau d'alcaloïdes.

4. Procédé selon la revendication 3 où ladite plante cultivée non alimentaire est une plante de type tabac **caractérisée en ce qu'**elle a un bas niveau de nicotine.

5. Procédé selon la revendication 4 où, dans ladite étape de transformation, ladite région régulatrice en 5' est choisie dans le groupe consistant en les promoteurs ubiquitaires et les promoteurs spécifiques de tissus.

6. Procédé selon la revendication 5 où, dans ladite étape de transformation, ledit acide nucléique inclut un motif de rétention du réticulum endoplasmique.

7. Procédé selon la revendication 6 où ledit acide nucléique inclut une séquence signal végétale.

8. Procédé selon la revendication 1 ou la revendication 2 où ledit tissu végétal comprenant IL-10 obtenu dans ladite étape d'obtention (étape iv) est destiné à l'administration orale à un animal.

9. Procédé de préparation d'IL-10 comprenant l'extraction d'IL-10 desdites plantes cultivées non alimentaires transformées selon la revendication 1
ou la revendication 2.

10. Composition de protéine préparée par le procédé selon la revendication 1 ou la revendication 2 comprenant IL-10 et des composants végétaux.

11. Composition de protéine selon la revendication 10 où la culture non alimentaire est le tabac.

12. Composition de protéine selon la revendication 10 où le tissu végétal est une feuille.

13. Composition de protéine préparée par le procédé selon la revendication 9 comprenant IL-10 et des composants végétaux solubles.

14. Procédé selon la revendication 1 où, dans ladite étape de transformation, ledit vecteur comprend aussi des séquences qui augmentent l'expression de ladite IL-10.

15. Procédé selon la revendication 1 où, dans ladite étape de transformation, ledit vecteur code un marqueur d'affinité.

16. Procédé selon la revendication 1 où ledit vecteur code aussi un site de clivage protéolytique.

17. Procédé selon la revendication 1 où ladite étape d'obtention comprend l'extraction d'IL-10 de ladite plante transformée pour produire un extrait brut.

18. Procédé selon la revendication 17 où ladite étape d'obtention comprend l'extraction d'IL-10 de ladite plante transformée et la purification partielle de ladite IL-10.

19. Procédé selon la revendication 4 où ladite plante de type tabac comprend de 0,2 % à 20 % d'alcaloïdes totaux de Delgold.

20. Utilisation de la composition de protéine selon la revendication 10
ou la revendication 13 dans la préparation d'un médicament pour le traitement d'un trouble médical chez un patient, où le trouble médical est la maladie de Crohn, l'arthrite induite par le collagène, le diabète, les affections liées à la coagulation sanguine, les affections médicales nécessitant la stimulation de cellules souches en monocytes/macrophages, les infections virales ou la régulation de réponses immunitaires.


### SEQUENCE LISTING

(1) GENERAL INFORMATION:
   (i) APPLICANT:
      (A) NAME: Jim Brandle
      (A) NAME Peter Davis
      (A) NAME: Kimberly Kenward
      (A) NAME: Rima Mensassa
      (A) NAME: Tony Jevmikar
      (A) NAME: Terry Delovitch
   (ii) TITLE OF INVENTION: Non-Food Crop Plant Bioreactor
   (iii) NUMBER OF SEQUENCES: 12
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30 (EPO)
(2) INFORMATION FOR SEQ ID NO: 1:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (iii) HYPOTHETICAL: NO
   (vi) ORIGINAL SOURCE:
      (A) ORGANISM: Tobacco mosaic virus
   (vii) IMMEDIATE SOURCE:
      (B) CLONE: 1091
   (viii) POSITION IN GENOME:
      (C) UNITS: bp
   (x) PUBLICATION INFORMATION:
      (A) AUTHORS: Richards, K
         Guilley, H
         Jonard, G
         Keith, G
      (B) TITLE: Leader sequence of 71 nucleotides devoid of G in tobacco mosaic virus RNA
      (C) JOURNAL: Nature
      (D) VOLUME: 267
      (F) PAGES: 548-550
      (G) DATE: 1977
   (x) PUBLICATION INFORMATION:
      (A) AUTHORS: Richards, K
         Guilley, H
         Jonard, G
         Hirth, L
      (B) TITLE: Nucleotide sequence at the 5' extremity of tobacco mosaic virus RNA
      (C) JOURNAL: Eur. J. Biochem.
      (D) VOLUME: 84
      (F) PAGES: 513-519
      (G) DATE: 1978
   (xi) SEQUENCE DESCRIPTION: SEQ ID NO: 1:
      GATCCTCGAG TATTTTTACA 20
(2) INFORMATION FOR SEQ ID NO: 2:
   (i) SEQUENCE CHARACTERISTICS:
      (A) LENGTH: 58 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (vii) IMMEDIATE SOURCE:
      (B) CLONE: 1309
   (xi) SEQUENCE DESCRIPTION: SEQ ID NO: 2:
      ACAATTACCA ACAACAACAA ACAACAAACA ACATTACAAT TACTATTTAC AATTACAA 58
(2) INFORMATION FOR SEQ ID NO: 3:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (iii) HYPOTHETICAL: NO
   (vii) IMMEDIATE SOURCE:
      (B) CLONE: 4361
   (xi) SEQUENCE DESCRIPTION: SEQ ID NO: 3:
      ACAATTACCA ACAACAACAA ACAACAAACA ACATTACAAT TACTATTTA 49
(2) INFORMATION FOR SEQ ID NO: 4:
   (i) SEQUENCE CHARACTERISTICS:
      (A) LENGTH: 74 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (vii) IMMEDIATE SOURCE:
      (B) CLONE: 1092
   (xi) SEQUENCE DESCRIPTION: SEQ ID NO: 4:
   TTGTAATTGT AAATAGTAAT TGTAATGTTG TTTGTTGTTT GTTGTTGTTG GTAATTGTTG 60
   TAAAAATACT CGAG 74
(2) INFORMATION FOR SEQ ID NO: 5:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (vii) IMMEDIATE SOURCE:
      (B) CLONE: 4362
   (xi) SEQUENCE DESCRIPTION: SEQ ID NO: 5:
      CAATTACAAT GGGATTTTTT CTCTTTTCAC AAATGCCAAG CTTTTTTCTT G 51
(2) INFORMATION FOR SEQ ID NO: 6:

(1) SEQUENCE CHARACTERISTICS:
   (A) LENGTH: 46 base pairs
   (B) TYPE: nucleic acid
   (C) STRANDEDNESS: single
   (D) TOPOLOGY: linear
      (ii) MOLECULE TYPE: other nucleic acid

   (A) DESCRIPTION: /desc = "oligonucleotide"
      (vii) IMMEDIATE SOURCE:
   (B) CLONE: 4363
      (xi) SEQUENCE DESCRIPTION: SEQ ID NO: 6:
         TCAGTACTCT TCTCTTATTC CTGATCATAT CTCACTCTTC GCATGC 46
(2) INFORMATION FOR SEQ ID NO: 7:
   (i) SEQUENCE CHARACTERISTICS:
      (A) LENGTH: 30 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: PR signal peptide cassette
   (xi) SEQUENCE DESCRIPTION: SEQ ID NO: 7:
   Met Gly Phe Phe Leu Phe Ser Gln Met Pro Ser Phe Phe Leu Val Ser
   1               5                   10                  15
   Thr Leu Leu Leu Phe Leu Ile Ile Ser His Ser Ser His Ala
               20                  25                  30
(2) INFORMATION FOR SEQ ID NO: 8:
   (i) SEQUENCE CHARACTERISTICS:
      (A) LENGTH: 56 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (vii) IMMEDIATE SOURCE:
      (B) CLONE: 4364
   (xi) SEQUENCE DESCRIPTION: SEQ ID NO: 8:
      GAGAAGAGTA CTGACAAGAA AAAAGCTTGG CATTTGTGAA AAGAGAAAAA ATCCCA 56
(2) INFORMATION FOR SEQ ID NO: 9:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (vii) IMMEDIATE SOURCE:
      (B) CLONE: 4365
   (xi) SEQUENCE DESCRIPTION: SEQ ID NO: 9:
      TAGCATGCGA AGAGTGAGAT ATGATCAGGA ATAA 34
(2) INFORMATION FOR SEQ ID NO: 10:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (vii) IMMEDIATE SOURCE:
      (A) LIBRARY: 3339
   (xi) SEQUENCE DESCRIPTION: SEQ ID NO: 10:
      TATTTTTTAC AACAATTACC AACAAC 26
(2) INFORMATION FOR SEQ ID NO: 11:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "oligonucleotide"
   (vii) IMMEDIATE SOURCE:
      (B) CLONE: 4708
   (xi) SEQUENCE DESCRIPTION: SEQ ID NO: 11:
      CAGCAGTCAT CTGCATACAG CAC 23
(2) INFORMATION FOR SEQ ID NO: 12:
   (i) SEQUENCE CHARACTERISTICS:
      (A) LENGTH: 1645 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: hIL-10-His-KDEL
   (xi) SEQUENCE DESCRIPTION: SEQ ID NO: 12:
   AAACCACAAG ACAGACTTGC AAAAGAAGGC ATGCACAGCT CAGCACTGCT CTGTTGCCTG 60
   GTCCTCCTGA CTGGGGTGAG GGCCAGCCCA GGCCAGGGCA CCCAGTCTGA GAACAGCTGC 120
   ACCCACTTCC CAGGCAACCT GCCTAACATG CTTCGAGATC TCCGAGATGC CTTCAGCAGA 180
   GTGAAGACTT TCTTTCAAAT GAAGGATCAG CTGGACAACT TGTTGTTAAA GGAGTCCTTG 240
   CTGGAGGACT TTAAGGGTTA CCTGGGTTGC CAAGCCTTGT CTGAGATGAT CCAGTTTTAC 300
   CTGGAGGAGG TGATGCCCCA AGCTGAGAAC CAAGACCCAG ACATCAAGGC GCATGTGAAC 360
   TCCCTGGGGG AGAACCTGAA GACCCTCAGG CTGAGGCTAC GGCGCTGTCA TCGATTTCTT 420
   CCCTGTGAAA ACAAGAGCAA GGCCGTGGAG CAGGTGAAGA ATGCCTTTAA TAAGCTCCAA 480
   GAGAAAGGCA TCTACAAAGC CATGAGTGAG TTTGACATCT TCATCAACTA CATAGAAGCC 540
   TACATGACAA TGAAGATACG AAACTGAGAC ATCAGGGTGG CGACTCTATA GACTCTAGGA 600
   CATAAATTAG AGGTCTCCAA AATCGGATCT GGGGCTCTGG GATAGCTGAC CCAGCCCCTT     660
   GAGAAACCTT ATTGTACCTC TCTTATAGAA TATTTATTAC CTCTGATACC TCAACCCCCA     720
   TTTCTATTTA TTTACTGAGC TTCTCTGTGA ACGATTTAGA AAGAAGCCCA ATATTATAAT     780
   TTTTTTCAAT ATTTATTATT TTCACCTGTT TTTAAGCTGT TTCCATAGGG TGACACACTA     840
   TGGTATTTGA GTGTTTTAAG ATAAATTATA AGTTACATAA GGGAGGAAAA AAAATGTTCT     900
   TTGGGGAGCC AACAGAAGCT TCCATTCCAA GCCTGACCAC GCTTTCTAGC TGTTGAGCTG     960
   TTTTCCCTGA CCTCCCTCTA ATTTATCTTG TCTCTGGGCT TGGGGCTTCC TAACTGCTAC    1020
   AAATACTCTT AGGAAGAGAA ACCAGGGAGC CCCTTTGATG ATTAATTCAC CTTCCAGTGT    1080
   CTCGGAGGGA TTCCCCTAAC CTCATTCCCC AACCACTTCA TTCTTGAAAG CTGTGGCCAG    1140
   CTTGTTATTT ATAACAACCT AAATTTGGTT CTAGGCCGGG CGCGGTGGCT CACGCCTGTA    1200
   ATCCCAGCAC TTTGGGAGGC TGAGGCGGGT GGATCACTTG AGGTCAGGAG TTCCTAACCA    1260
   GCCTGGTCAA CATGGTGAAA CCCCGTCTCT ACTAAAAATA CAAAAATTAG CCGGGCATGG    1320
   TGGCGCGCAC CTGTAATCCC AGCTACTTGG GAGGCTGAGG CAAGAGAATT GCTTGAACCC    1380
   AGGAGATGGA AGTTGCAGTG AGCTGATATC ATGCCCCTGT ACTCCAGCCT GGGTGACAGA    1440
   GCAAGACTCT GTCTCAAAAA AATAAAAATA AAAATAAATT TGGTTCTAAT AGAACTCAGT    1500
   TTTAACTAGA ATTTATTCAA TTCCTCTGGG AATGTTACAT TGTTTGTCTG TCTTCATAGC    1560
   AGATTTTAAT TTTGAATAAA TAAATGTATC TTATTCACAT CAAAAAAAAA AAAAAAAAAA    1620
   CTCGAGGGGG GGCCGGTACC CAATG                                          1645